# EUROPEAN PATENT SPECIFICATION

(11) **EP 1 713 452 B1**
(45) Date of publication and mention of the grant of the patent: **24.03.2010**
(21) Application number: 05707079.9
(22) Date of filing: 28.01.2005
(51) Int. Cl.: A61K 9/20, A61K 31/404, A61P 9/12

(54) **SUSTAINED RELEASE PHARMACEUTICAL COMPOSITION OF INDAPAMIDE**
PHARMAZEUTISCHE INDAPAMID-ZUSAMMENSETZUNG MIT VERZÖGERTER FREISETZUNG
COMPOSITION PHARMACEUTIQUE D'INDAPAMIDE A LIBERATION PROLONGEE

(30) Priority: 30.01.2004 DE 102004005009
(43) Date of publication of application: 25.10.2006
(73) Proprietor: KRKA, tovarna zdravil, d.d., Novo mesto, 8501 Novo mesto (SI)
(72) Inventor: KRAMAR, Andrejka, 8000 Novo mesto (SI); VRECER, Franc;, 8351 Straza pri Novem mestu (SI); PETRISIC, Irena, 8280 Brestanica (SI); PERC, Stanka, 8344 Vinica pri Crnomlju (SI)
(74) Representative: UEXKÜLL & STOLBERG
(86) International application number: PCT/EP2005/000886
(87) International publication number: WO 2005/074884

(56) References cited:
- EP-A- 0 519 820
- EP-A- 1 057 479
- WO-A-2004/002475
- CUI ET AL: "Development of indapamide sustained-release tablets and study on its release mechanism" CA, 2003, XP002332372

## Description

### FIELD OF THE INVENTION

The present invention relates to a sustained release pharmaceutical composition of indapamide which can be in the form of a tablet obtainable through a direct compression process.

### BACKGROUND OF THE INVENTION

Sustained release formulations allow the possibility of reducing dosage regimens for drugs, especially those administered orally to patients. The advantages of reduced dosage regimens for the patients are improved convenience, better assurance of compliance, reduction of severity and frequency of side effects, as such formulations maintain substantially constant blood levels and avoid the fluctuations associated with the conventional immediate release formulations administered more than once a day.

Sustained release can inter alia be achieved by using specific materials for forming a hydrophilic or lipophilic matrix within the formulation or by specific coatings on the formulation.

Two general procedures can be used for obtaining hydrophilic matrix tablets. The first process is a direct compression process and involves the direct mixing of the dry components of the desired formulation, followed by a compression step to manufacture tablets. For such a process, excipients with good compression properties are necessary and a very good homogeneity of the powder mixture is mandatory. The second process is wet granulation. In this case, the powder obtained following the initial mixing of the components is granulated using proper quantities of granulation liquid. After drying and the addition of other excipients, if necessary, the granulate has normally good compressibility properties.

Sustained release formulations of indapamide are known.

WO2004/002475 A1 discloses sustained release tablets comprising indapamide, lactose monohydrate, copovidone, and hypromellose.

EP-B-519 820 discloses matrix tablets for sustained release of indapamide, wherein the sustained release is controlled by the use of specific amounts of methylhydroxypropyl cellulose and polyvidone. The process for preparation of the tablet includes a wet granulation step wherein the indapamide, polyvidone and lactose are combined with an aqueous-alcoholic granulation liquid to yield granules, which are then dried and mixed with the methylhydroxypropyl cellulose. Finally, lubricant is added and the mixture is compressed to give tablets.

A similar process for preparing indapamide tablets is described by G. Damien et al. in a review article in Clin. Pharmacokinetic 1999, 37 Suppl. 1, pages 13-19 which differs from that described in EP-B-519 820 by the use of water as granulation liquid.

However, these prior art processes result in tablets showing high fluctuations in terms of their content which for pharmaceutical preparations administering active ingredients to the human body is highly undesirable. Apart from this low uniformity as to the content of the tablets, such tablets also suffer from substantial changes in the dissolution profile when freshly prepared tablets are compared with those which have been stored for some time. It is clear that stability of the dissolution profile even after storage is a vital factor influencing the quality of a pharmaceutical composition.

It is, therefore, an object of the present invention to provide a pharmaceutical composition which is not only able to provide a sustained release of indapamide, but also avoids the above mentioned problems of the prior art compositions of undesirably high variations as to the content uniformity and the dissolution profile.

This object is surprisingly solved by the sustained release pharmaceutical composition according to claim 1 to 13. The invention is also directed to the process for preparing the composition according to claim 14 to 15.

### DETAILED DESCRIPTION OF THE INVENTION

The sustained release pharmaceutical composition according to the invention comprises
(a) indapamide or a hydrate or a solvate or a salt thereof,
(b) a mixture of at least one oligosaccharide and powdered cellulose as direct compression excipient, and
(c) cellulose derivative as hydrophilic matrix former.

It has surprisingly been shown that by the use of the specific direct compression excipient (b) in combination with the specific hydrophilic matrix former (c) a composition is obtained which can be processed to dosage forms, such as tablets, by direct compression methods without need of using wet granulation steps. Even though no wet granulation step is used, the dosage uniformity of the obtained composition is excellent. This factor is surprising since G. Damien et al. states in the afore-mentioned article that a high dosage uniformity can hardly be obtained by direct compression.

Moreover, it has also been shown and is in more detail described in the examples that the pharmaceutical composition of indapamide according to the invention shows only very small differences in terms of dissolution profile when comparing freshly prepared compositions with those which have stored for some time in a humid atmosphere.

Thus, the pharmaceutical composition according to the invention apparently combines the advantageous property normally attributed to a wet granulation process, namely a high homogeneity, with the advantages of a direct compression process, namely the avoiding of granulation liquids which may be the primary cause of instability of dosage forms and moreover require an additional drying step to remove the granulation liquid in order to give the final dosage form.

The indapamide used in the pharmaceutical composition of the invention can also be present in form of a hydrate thereof. The water content of a hydrate depends on the humidity level of the atmosphere and can normally be up to 3% in case of the hemihydrate. A useful hydrate is indapamide hemihydrate.

The pharmaceutical composition according to the invention can be in various forms preferably those which allow oral administration. The composition is particularly preferred in form of tablets. Any form which is for administration by the oral route usually comprises indapamide or indapamide hydrate or a solvate or a salt thereof in an amount of 1.5 to 5 mg, based on indapamide.

It has further been shown that pharmaceutical compositions are preferred which comprise indapamide or a hydrate thereof in form of particles having specific sizes. The reason is that the particle size may have an influence on content uniformity as well on the release profile of the compositions. Particles of indapamide or a hydrate thereof are preferred which have a particle size distribution of 90% of the particles < 80µm, preferably 90% of the particles < 70µm, and 50% of the particles < 40µm, preferably 50% of the particles < 30pm, as determined by laser diffraction analysis.

The specific direct compression excipient (b) of the composition according to the invention is mainly imparting a good flowability and compressibility to the composition and therefore allows its easy and satisfactory direct compression. This direct compression excipient is a mixture of at least one oligosaccharide and powdered cellulose. Useful oligosaccharides are compounds having 2 to 6 and preferably 2 to 3 monosaccharide moieties.

The oligosaccharide is preferably selected from the group of lactose and sucrose. A particularly useful lactose is α-lactose and preferably α-lactose monohydrate.

The polysaccharide is powdered cellulose, sometimes also referred to as cellulose powder.

A preferred mixture used as a direct compression excipient (b) comprises 70 to 80% by weight of oligosaccharide and 20 to 30% by weight of powdered cellulose, based on the weight of mixture.

An even more preferred mixture (b) comprises 73 to 77% by weight of oligosaccharide and 23 to 27% by weight of powdered cellulose.

The most preferred mixture (b) comprises about 75% by weight of oligosaccharide and about 25% by weight of powdered cellulose.

The hydrophilic matrix former (c) included in the composition according to the invention is a cellulose derivative which mainly serves to achieve the desired dissolution profile of indapamide so that a sustained release of the active ingredient is obtained.

This cellulose derivative can be one cellulose derivative or a mixture thereof. It is preferred that this cellulose derivative is selected from the group consisting of hydroxypropyl cellulose, hydroxyethyl cellulose, hydroxypropylmethyl cellulose and methyl cellulose. The preferred cellulose derivative is hydroxypropylmethyl cellulose.

The hydroxypropylmethyl cellulose is commercially available in various viscosity grades. It has been shown that a hydroxypropylmethyl cellulose is particularly advantageous which has a viscosity of 1.000 to 100.000 cps. The most preferred cellulose derivative is a hydroxypropylmethyl cellulose having a viscosity of 1.000 to 15.000 cps.

A composition according to the invention is preferred which comprises
(a) 0.5 to 5.0% by weight of indapamide or a hydrate or a solvate or a salt thereof, based on indapamide,
(b) 40 to 80% by weight of direct compression excipient, and
(c) 10 to 50% by weight of hydrophilic matrix former.

Preferably, the amount of indapamide in a composition according to the invention is 0.5 to 1.5% by weight and more preferably 0.5 to 1.0% by weight. In particular, the composition comprises about 0.75% by weight of indapamide or a hydrate or a solvate or a salt thereof, based on indapamide.

Apart from the components (a), (b) and (c), the composition according to the invention, which is preferably in form of tablets, may also include other excipients and additives conventional in the art, like binders, lubricants, glidants and combinations thereof.

The amounts of these additional components are for binder 1 to 10%, preferably 2 to 8% by weight, for lubricant 0.2 to 3%, preferably 0.2 to 1.0% by weight and glidant 0.1 to 0.5%, preferably 0.1 to 0.3% by weight.

The invention also provides a process for preparing the composition according to the invention which comprises
(i) dry mixing of indapamide or a hydrate or a solvate or a salt thereof (a), direct compression excipient (b) and hydrophilic matrix former (c) and optional additional components, and
(ii) optionally compressing the obtained mixture to the desired form.

In a preferred embodiment in step (i), the components (a), (b) and (c) are dry mixed with optionally present binder and glidant and to this mixture lubricant is added and the mixture is homogenised. Finally, the homogenised mixture is compressed into the desired form, preferably tablets.

Any suitable solid dosage form of the composition according to the invention may be coated with usual coating substances, such as polymers. Such coatings may provide an additional or modifying controlled release effect or may serve to mask the taste or odour or to further improve the stability of the dosage form.

It has surprisingly been shown that the combination of indapamide or a hydrate thereof with the specific direct compression excipient (b) and the specific hydrophilic matrix former (c) leads to compositions which do not suffer from the problems of inacceptably high variations in content uniformity and stability of the dissolution profiles. It is also very beneficial that the compositions according to the invention can be directly compressed in the dry state into the desired form, such as tablets, without the need for a wet granulation step to ensure high homogeneity. Nevertheless, the tablets according to the invention show only slight variations in terms of their indapamide content and they also have dissolution profiles which do not differ significantly when comparing freshly prepared compositions with those which have been kept on storage in a humid atmosphere. Finally, in process controls have shown that the relative standard deviation as to the weight variation of tablets according to the invention are usually in the range of only 0.8 to 1.5% on a tableting machine operating at various compression speeds. Consequently, the compositions according to the invention are showing substantial advantages over the sustained release formulations of indapamide known in the

### prior art.

The invention is in the following illustrated in more detail by reference to examples.

### EXAMPLES

### EXAMPLES 1 TO 4

### Process for manufacturing tablets

Tablets were manufactured using a direct compression procedure (examples 3 and 4) and for comparative purposes using a wet granulation procedure (examples 1 and 2, comparison).

For examples 1 and 2 (wet granulation procedure) indapamide, polyvidone (binder) and lactose were screened. The screened materials were dry blended and then granulated with water in example 1 or a water/ethanol solution (50%/50% by weight) in example 2 in a conventional manner. The obtained wet granulates were dried until the granulate has a moisture content of less than 2% by weight of water. The dried granules were screened and mixed with hydroxypropylmethyl cellulose, magnesium stearate (lubricant) and silica, colloidal anhydrous (glidant). The resulting mixture was then compressed using a rotary tableting machine to give tablets.

In examples 3 and 4 (direct compression procedure), indapamide and all the excipients and additional components were screened. The screened materials, with the exception of magnesium stearate (lubricant), were dry blended. The lubricant was then added to the resulting mixture and the mixture was homogenised. The final mixture was compressed using a rotary tableting machine to give tablets.

### Composition of tablets

The compositions of the tablets of examples 1 to 4 were as follows:

| Example | 1 (comparison) wet granulation | 2 (comparison) wet granulation | 3 direct compression | 4 direct compression |
|---|---|---|---|---|
| Ingredient | mg/ tablet | mg/ tablet | mg/ tablet | mg/tablet |
| Indapamide | 1.5 | 1.5 | 1.5 | 1.5 |
| Lactose | 124.50 | 124.50 | - | - |
| Povidone | 8.6 | 8.6 | 8.6 | - |
| Water* | 16.0 | 8.0 | - | - |
| Ethanol (96 %)* | - | 8.0 | - | - |
| Hydroxypropylmethylcellulose | 64.0 | 64.0 | 64.0 | 64.0 |
| Mixture of 75% by weight α-lactose monohydrate and 25% by weight cellulose powder | - | - | 124.5 | 133.1 |
| Silica, colloidal anhydrous | 0.4 | 0.4 | 0.4 | 0.4 |
| Magnesium stearate | 1.0 | 1.0 | 1.0 | 1.0 |

| | | | | |
|---|---|---|---|---|
| * evaporates during the process | | | | |

### The content uniformity of the tablets

The tablets obtained in example 3 and in comparative examples 1 and 2 were analysed for their content of indapamide. This was done by a HPLC method.

A liquid chromatographic system with binary pump and variable wavelength detector set at 254 nm was used. The mobile phase consisted essentially of a mixture of methanol and acetonitrile. A stainless-steel column Symmetry C18, packed with 3.5 µm particles was used for separation. 10 µm of Standard solution and Sample solution with a working concentration of about 0.06 mg/ml of indapamide in a mixture of equal volumes of methanol and acetonitrile were injected. 30 tablets were chosen for the analyses. From these 30 tablets 10 tablets were placed into a suitable volumetric flask and treated all in the same way. The percentage of indapamide relative to the stated amount for each tablet was calculated.

The result of these analysis are given in the following table

| Example 1 (wet granulation) (%) | Example 2 (wet granulation) (%) | Example 3 (dry compression) (%) |
|---|---|---|
| 100.3 | 95.3 | 96.7 |
| 97.4 | 98.9 | 97.1 |
| 99.2 | 104.8 | 98.9 |
| 94.7 | 98.4 | 99.4 |
| 109.3 | 96.3 | 97.0 |
| 109.0 | 99.0 | 97.8 |
| 108.4 | 92.9 | 98.7 |
| 97.0 | 96.6 | 97.9 |
| 97.0 | 99.2 | 98.7 |
| 98.7 | 95.2 | 96.7 |
| Average 101.4 | 97.6 | 97.9 |
| **RSD* 5.2** | **3.3** | |
| **5.2** | | **1.1** |

| | | |
|---|---|---|
| *RSD - relative standard deviation | | |

As can be seen from the above data, the relative standard deviation for the content of the tablets according to example 3 was only 1.1. The relative standard deviation for comparative examples 1 and 2 was much higher, namely 5.2 and 3.3, respectively. This shows that the tablets according to the invention do not suffer from high variations in terms of their content of indapamide like the conventional tablets.

### Dissolution tests of the tablets

Tablets prepared according to example 3 and according to example 1 (comparative) were subjected to a dissolution test using freshly prepared tablets and tablets which have been put on storage for 1 month in an atmosphere of 74% relative humidity at 40°C. These drastic conditions were selected to simulate storage over a long period of time which in practice usually occurs.

The dissolution test was carried out in 500ml phosphate buffer (USP) having a pH value of 6.8 using USP paddle apparatus II at 50 rpm and 37°C. The table below shows cumulative % of release of indapamide at specific points in time.

| Formulation/Time (h) | Example 1 Fresh sample | Example 1 After 1month (40°C/75%RH) | Example 3 Fresh sample | Example 3 After 1month (40°C/75%RH) |
|---|---|---|---|---|
| 1 | 4 | 3 | 8 | 7 |
| 2 | 8 | 6 | 13 | 11 |
| 4 | 15 | 12 | 21 | 19 |
| 6 | 22 | 19 | 28 | 26 |
| 8 | 30 | 26 | 35 | 32 |
| 10 | 36 | 32 | 41 | 39 |
| 12 | 43 | 38 | 47 | 44 |
| 24 | 79 | 68 | 79 | 73 |

The above data show that the differences between the fresh sample and the sample stored for 1 month are significantly smaller for the tablets according to the invention of example 3 than for those of comparative example 1. For example 3 the fresh sample released 79% and the stored sample 73% of indapamide after 24 hours. In contrast to this, for example 1 the freshly prepared sample had also released 79%, but the stored sample only 68% of indapamide.

It can therefore be concluded that the tablets according to the invention have a higher stability than the conventional sustained release tablets.

## Claims

1. Sustained release pharmaceutical composition comprising
(a) indapamide or a hydrate or a solvate or a salt thereof,
(b) a mixture of at least one oligosaccharide and powdered cellulose as direct compression excipient, and
(c) cellulose derivative as hydrophilic matrix former.

2. Composition according to claim 1, wherein the oligosaccharide is selected from lactose and sucrose.

3. Composition according to claim 2, wherein the lactose is α-lactose, preferably α-lactose monohydrate.

4. Composition according to any one of claims 1 to 3, wherein the mixture (b) comprises
70 to 80% by weight of oligosaccharide and
20 to 30% by weight of powdered cellulose,
based on the weight of the mixture.

5. Composition according to claim 4, wherein the mixture (b) comprises
73 to 77% by weight of oligosaccharide and
23 to 27% by weight of powdered cellulose.

6. Composition according to any one of claims 1 to 5, wherein the cellulose derivative is selected from hydroxypropyl cellulose, hydroxypropylmethyl cellulose and methyl cellulose.

7. Composition according to claim 6, wherein the hydroxypropylmethyl cellulose has a viscosity of 1000 to 15000cps.

8. Composition according to any one of claims 1 to 7, comprising
(a) 0.5 to 5.0% by weight of indapamide or a hydrate or a solvate or a salt thereof,
(b) 40 to 80% by weight of direct compression excipient, and
(c) 10 to 50% by weight of hydrophilic matrix former.

9. Composition according to claim 8, comprising 0.5 to 1.5% by weight of indapamide.

10. Composition according to any one of claims 1 to 9, comprising indapamide hemihydrate.

11. Composition according to any one of claims 1 to 10, which is in form of a tablet.

12. Composition according to any one of claims 1 to 11, comprising 1 to 10% by weight of a binder, 0.2 to 3% by weight of a lubricant and 0.1 to 0.5% by weight of a glidant.

13. Composition according to any one of claims 1 to 12, wherein the indapamide or the hydrate or the solvate or the salt thereof has a particle size distribution of 90% of the particles < 70 µm and 50% of the particles < 30 µm.

14. Process for preparing the composition according to any one of claims 1 to 13, which comprises
(i) dry mixing of indapamide or a hydrate or a solvate or a salt thereof (a), direct compression excipient (b) and hydrophilic matrix former (c) and optional additional components, and
(ii) optionally compressing the obtained mixture to the desired form.

15. Process according to claim 14, wherein in step (i) the components (a), (b) and (c) are dry mixed with optionally present binder and glidant and to this mixture lubricant is added and the mixture is homogenised.

## Patentansprüche

1. Pharmazeutische Zusammensetzung mit verlängerter Freisetzung, die
(a) Indapamid oder ein Hydrat oder ein Solvat oder ein Salz davon,
(b) eine Mischung von mindestens einem Oligosaccharid und Cellulosepulver als Direktverpressungshilfsstoff, und
(c) ein Cellulosederivat als hydrophilen Matrixbildner
enthält.

2. Zusammensetzung nach Anspruch 1, in der das Oligosaccharid aus Lactose und Saccharose ausgewählt ist.

3. Zusammensetzung nach Anspruch 2, in der die Lactose α-Lactose, vorzugsweise α-Lactosemonohydrat ist.

4. Zusammensetzung nach einem der Ansprüche 1 bis 3, in der die Mischung (b) bezogen auf das Gewicht der Mischung
70 bis 80 Gew.-% Oligosaccharid und
20 bis 30 Gew.-% Cellulosepulver
enthält.

5. Zusammensetzung nach Anspruch 4, in der die Mischung (b)
73 bis 77 Gew.-% Oligosaccharid und
23 bis 27 Gew.-% Cellulosepulver

6. Zusammensetzung nach einem der Ansprüche 1 bis 5, in der das Cellulosederivat aus Hydroxypropylcellulose, Hydroxypropylmethlycellulose und Methylcellulose ausgewählt ist.

7. Zusammensetzung nach Anspruch 6, in der die Hydroxypropylmethylcellulose eine Viskosität von 1.000 bis 15.000 cps aufweist.

8. Zusammensetzung nach einem der Ansprüche 1 bis 7, die
(a) 0,5 bis 5,0 Gew.-% Indapamid oder ein Hydrat oder ein Solvat oder ein Salz davon,
(b) 40 bis 80 Gew.-% eines Direktverpressungshilfsstoffs, und
(c) 10 bis 50 Gew.-% eines hydrophilen Matrixbildners
enthält.

9. Zusammensetzung nach Anspruch 8, die 0,5 bis 1,5 Gew.-% Indapamid enthält.

10. Zusammensetzung nach einem der Ansprüche 1 bis 9, die Indapamidhemihydrat enthält.

11. Zusammensetzung nach einem der Ansprüche 1 bis 10, die in Form einer Tablette vorliegt.

12. Zusammensetzung nach einem der Ansprüche 1 bis 11, die 1 bis 10 Gew.-% eines Bindemittels, 0,2 bis 3 Gew.-% eines Schmiermittels und 0,1 bis 0,5 Gew.-% eines Fließregulierungsmittels enthält.

13. Zusammensetzung nach einem der Ansprüche 1 bis 12, in der das Indapamid oder das Hydrat oder das Solvat oder das Salz davon eine Partikelgrößenverteilung von 90% der Partikel < 70 µm und 50% der Partikel < 30 µm aufweist.

14. Verfahren zur Herstellung der Zusammensetzung nach einem der Ansprüche 1 bis 13, bei dem
(i) Indapamid oder ein Hydrat oder ein Solvat oder ein Salz davon (a), Direktverpressungshilfsstoff (b) und hydrophiler Matrixbildner (c) und gegebenenfalls weitere Bestandteile trockengemischt werden, und
(ii) gegebenenfalls die erhaltene Mischung zu der gewünschten Form verpresst wird.

15. Verfahren nach Anspruch 14, bei dem in Schritt (i) die Komponenten (a), (b) und (c) mit gegebenenfalls vorhandenem Bindemittel und Fließregulierungsmittel trockengemischt werden und zu dieser Mischung Schmiermittel zugegeben wird und die Mischung homogenisiert wird.

## Revendications

1. Composition pharmaceutique à libération prolongée, comprenant :
a) de l'indapamide, ou un hydrate, un solvat ou un sel d'indapamide,
b) un mélange d'au moins un oligosaccharide et de cellulose en poudre, en guise d'excipient pour compression directe,
c) et un dérivé de cellulose, en tant que formateur de matrice hydrophile.

2. Composition conforme à la revendication 1, dans laquelle l'oligosaccharide est choisi parmi du lactose et du saccharose.

3. Composition conforme à la revendication 2, dans laquelle le lactose est de l'α-lactose, et de préférence, du monohydrate d'α-lactose.

4. Composition conforme à l'une des revendications 1 à 3, dans laquelle le mélange (b) est constitué de 70 à 80 % en poids d'oligosaccharide et de 20 à 30 % en poids de cellulose en poudre, en pourcentages rapportés au poids du mélange.

5. Composition conforme à la revendication 4, dans laquelle le mélange (b) est constitué de 73 à 77 % en poids d'oligosaccharide et de 23 à 27 % en poids de cellulose en poudre.

6. Composition conforme à l'une des revendications 1 à 5, dans laquelle le dérivé de cellulose est choisi parmi de l'hydroxypropyl-cellulose, de l'hydroxypropyl-méthyl-cellulose et de la méthyl-cellulose.

7. Composition conforme à la revendication 6, dans laquelle l'hydroxypropyl-méthyl-cellulose présente une viscosité de 1000 à 15000 centi-poises.

8. Composition conforme à l'une des revendications 1 à 7, qui comprend :
a) de 0,5 à 5,0 % en poids d'indapamide, ou d'un hydrate, d'un solvat ou d'un sel d'indapamide,
b) de 40 à 80 % en poids de l'excipient pour compression directe,
c) et de 10 à 50 % en poids du formateur de matrice hydrophile.

9. Composition conforme à la revendication 8, qui comprend de 0,5 à 1,5 % en poids d'indapamide.

10. Composition conforme à l'une des revendications 1 à 9, qui comprend de l'hémihydrate d'indapamide.

11. Composition conforme à l'une des revendications 1 à 10, qui se présente sous la forme de comprimé.

12. Composition conforme à l'une des revendications 1 à 11, qui comprend de 1 à 10 % en poids d'un liant, de 0,2 à 3 % en poids d'un lubrifiant et de 0,1 à 0,5 % en poids d'un agent de glissement.

13. Composition conforme à l'une des revendications 1 à 12, dans laquelle l'indapamide ou l'hydrate, le solvat ou le sel d'indapamide présente une distribution de tailles de particules où 90 % des particules ont une taille inférieure à 70 µm et 50 % des particules ont une taille inférieure à 30 µm.

14. Procédé de préparation d'une composition conforme à l'une des revendications 1 à 13, lequel procédé comporte les étapes suivantes :
i) mélanger à sec l'indapamide ou l'hydrate, le solvat ou le sel d'indapamide (a), l'excipient pour compression directe (b) et le formateur de matrice hydrophile (c), ainsi que, en option, des composants supplémentaires ;
ii) et en option, comprimer le mélange obtenu, de manière à le mettre sous la forme voulue.

15. Procédé conforme à la revendication 14, dans lequel, dans l'étape (i), on mélange à sec les composants (a), (b) et (c), ainsi que le liant et l'agent de glissement optionnels, on ajoute un lubrifiant au mélange ainsi obtenu et l'on homogénéise le tout.
